# EUROPÄISCHE PATENTSCHRIFT

(11) **EP 0 534 357 B1**
(45) Veröffentlichungstag und Bekanntmachung des Hinweises auf die Patenterteilung: **18.12.1996**
(21) Anmeldenummer: 92116160.0
(22) Anmeldetag: 22.09.1992
(51) Int. Cl.: C07C 333/16, C10M 135/18

(54) **Neue Wolfram-hexa-(dialkyldithiocarbamate) und Verfahren zu ihrer Herstellung**
New tungsten-hexa-(dialkyldithiocarbamates) and process for their preparation
Tungstène-hexa-(dialcoyl dithiocarbamates) nouveaux et procédé pour leur préparation

(30) Priorität: 25.09.1991 DE 4131921
(43) Veröffentlichungstag der Anmeldung: 31.03.1993
(73) Patentinhaber: Dr. Spiess Chemische Fabrik GmbH & Co., D-67271 Kleinkarlbach (DE)
(72) Erfinder: Spiess, Wolfram, Dr., W-6718 Grünstadt (DE); Franke, Friedrich, Dr., W-6718 Grünstadt (DE); Himmerlreich, Rolf, Verstorben (DE)
(74) Vertreter: Eggert, Hans-Gunther, Dr.

(56) Entgegenhaltungen:
- WO-A-87/05045
- US-A- 2 258 847
- US-A- 3 244 627
- F.A. COTTON/G. WILKINSON "ANORGANISCHE CHEMIE", 3.AUFLAGE, VERLAG CHEMIE, WEINHEIM, 1980, SEITEN 1016-1019
- HOUBEN-WEYL (METHODEN DER ORGANISCHEN CHEMIE), 4.AUFLAGE, BAND IX "SCHWEFEL-SELEN-, TELLUR-VERBINDUNGEN", GEORG THIEME VERLAG, STUTTGART, 1955, SEITEN 824-827

## Beschreibung

Nach den bekannten technischen Verfahren erfolgt die Herstellung der Schwermetalldithiocarbamate entweder durch doppelte Umsetzung von Alkali- bzw. Ammoniumdithiocarbamaten mit Schwermetallhalogeniden oder durch Umsetzung von Metalloxiden mit Aminen und Schwefelkohlenstoff.

Die Umsetzung mit Metalloxiden führt bei den bekannten technischen Methoden nur bei Wertigkeiten des Metalls von 2 - 4 zu oxidfreien Dialkyldithiocarbamaten der allgemeinen Formel 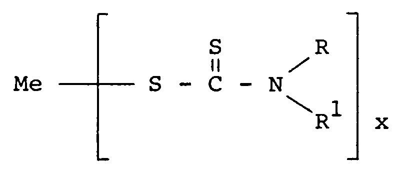
wobei x die Valenz des eingesetzten Metalls darstellt.

Umsetzungen mit Oxiden von Metallen höherer Wertigkeit führen zur Bildung von oxidhaltigen Dialkyldithiocarbamatgemischen der allgemeinen Formel 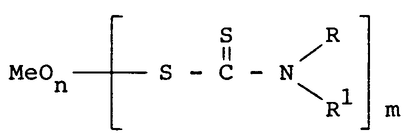 n, m = 1 - 2

Die oxidfreien Dithiocarbamate des Wolframs sind bisher nicht beschrieben.

So betrifft die US-A-3,244,627 Schwermetall-bis(N,N-diorganodithiocarbamate) der allgemeinen Formel (R'R)-N-(S)C-S-N-S-(S)C-N(RR'), bei denen R und R' unabhängig voneinander Kohlenstoffreste, welche frei von olefinischen und azetylenischen Verunreinigungen sind und 1 bis 12 Kohlenstoffatome aufweisen ... usw. und N ein **divalentes** Schwermetallion ist. Als Schwermetallion kommt beispielsweise **divalentes** Zink, Cadmium, Quecksilber ... Wolfram ... in Betracht. Die **divalenten** Schwermetallionen der Gruppe I - IV und VII - VIII sind bevorzugt. Besonders bevorzugt ist das Wolfram- **bis**(N,N-dimethyldithiocarbamat) benannt. Besagte Diorganodithiocarbamate werden hergestellt, beispielsweise durch Umsetzung eines sekundären Amins mit Schwefelkohlenstoff und Ammoniumhydroxyd oder Alkalimetallhydroxyden zur Erzeugung des Ammonium- oder Alkalimetallsalzes und nachfolgende Umsetzung von **zwei Molen** diesen Salzes mit **einem Mol** eines Metallsalzes, beispielsweise eines Acetates. Hieraus kann gefolgert werden, daß Gegenstand dieser Druckschrift die Bereitstellung von **Schwermetallbis(N,N-diorganodithiocarbamaten)** ist, welche als Additive für Polyvinylether eingesetzt werden.

Gegenstand der Erfindung sind Wolfram-hexa(dialkyldithiocarbamate) der allgemeinen Formel I 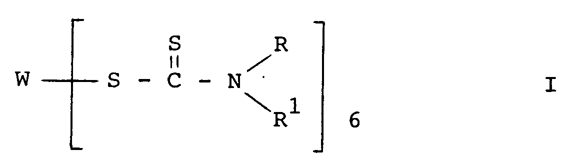
in der R und R¹ gleiche oder verschiedene, verzweigte oder unverzweigte, gesättigte oder ungesättigte Alkylreste mit 1 - 18 Kohlenstoffatomen sein können.

Gegenstand der Erfindung ist ferner ein Verfahren zur Herstellung der erfindungsgemäßen Verbindungen der allgemeinen Formel I.

Es wurde nun gefunden, daß durch Umsetzung von Alkalimetall-dialkyl-dithiocarbamaten mit Wolframhexachlorid definierte Verbindungen der allgemeinen Formel I herzustellen sind. Da diese Verbindungen hervorragende Eigenschaften als Öladditive besitzen, stellt die Herstellung und Bereitstellung der erfindungsgemäßen Verbindungen eine wesentliche Verbesserung des Standes der Technik dar, zumal durch die Verbesserung der Ölqualität eine umweltverträglichere Nutzung der Hydraulik- oder Getriebeöle gegeben ist.

Die Herstellung erfolgt nach folgendem Reaktionsschema 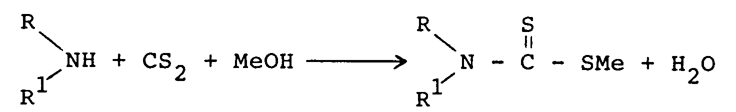 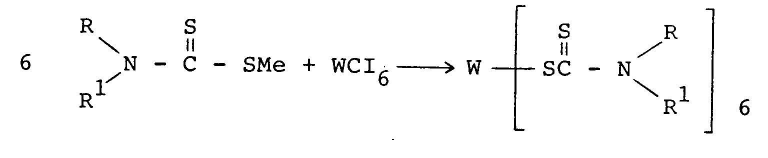
- R und R¹ :: obige Bedeutung
- Me :: Na, K, NH₄

Die Herstellung der Alkalidialkyldithiocarbamate ist im Prinzip bekannt. Dabei wird im allgemeinen folgendermaßen verfahren:
In wasserfreiem Methanol wird festes Natriumhydroxid gelöst und zu dieser Lösung das Dialkylamin gegeben.
Die Bildung des Natriumdialkyldithiocarbamates erfolgt durch Zugabe von Schwefelkohlenstoff. Alle Reaktionskomponenten werden in stöchiometrischen Mengen eingesetzt, wobei Schwefelkohlenstoff vorzugsweise mit einem 10%igen Überschuß gehandhabt wird. Die Mischung wird 1 - 3 Stunden bei Temperaturen von 10 - 50°C, vorzugsweise bei etwa 25°C, gerührt.
Das so hergestellte Natrium-dialkyldithiocarbamat kann in Lösung sofort weiterverarbeitet werden und dient als Ausgangsprodukt für die Umsetzung mit Wolframhexachlorid.
Stöchiometrische Mengen des Wolframhexachlorids werden in wasserfreiem Methanol gelöst und mit der Natriumdialkyldithiocarbamat zur Umsetzung gebracht. Die Reaktion erfolgt bei Temperaturen von 10 - 90°C, vorzugsweise bei etwa 30°C.

Die Reaktion läuft in beiden Stufen schwach exotherm ab, so daß eine zusätzliche Energiezuführung nicht erforderlich ist.

Nach einer Umsetzungsdauer von 2 Stunden wird vom ausgefallenen Alkalichlorid abfiltriert und das Methanol im Vakuum bei einer Temperatur von 50°C abdestilliert. Da so erhaltene braune Öl wird gegebenenfalls nochmals von Natriumchloridresten durch Filtration getrennt.

An Stelle des Methanols können auch andere wasserfreie Lösungsmittel wie Ethanol, Butanol, Isopropanol, Dioxan, Tetrahydrofuran oder Ethylenglykolmonoalkylether eingesetzt werden.

Die folgenden Beispiele zur Herstellung der erfindungsgemäßen Verbindungen sollen die Erfindung beschreiben, ohne sie einzuschränken.

### Beispiel 1

### Wolfram-hexa(di-amyldithiocarbamat)

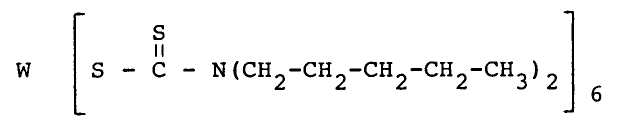

- Summenformel:: C₆₆H₁₃₂N₂S₁₂W
- Molekulargewicht:: 1578,379

Zu 8 Liter wasserfreiem Methanol werden 0,576 kg granuliertes Ätznatron zugegeben und gerührt bis alles in Lösung gegangen ist. Dazu werden 2,26 kg Diamylamin gegeben und innerhalb von zwei Stunden 0,88 Liter Schwefelkohlenstoff eingetragen. Es wird noch 3 Stunden bei 25°C nachgerührt und anschließend eine Lösung von 0,952 kg Wolframhexachlorid in 2,4 Liter wasserfreiem Methanol innerhalb von 1 Stunde zugegeben und noch 3 Stunden weitergerührt.

Vom Niederschlag wird abfiltriert und aus dem Filtrat wird Methanol abdestilliert und der Rückstand im Vakuum getrocknet. Es wurde ein viskoses braunes Produkt erhalten.
- Ausbeute:: 88 % der Theorie
- Gehalt:: über Stickstoffbestimmung―100 %

### Beispiel 2

### Wolfram-hexa-(di-2-ethylhexyl-dithiocarbamat)

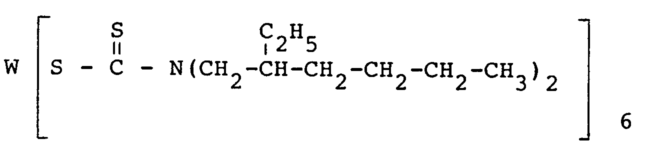

- Summenformel:: C₁₀₂H₂₀₄N₆S₁₂W
- Molekulargewicht:: 2083,34

Zu 10 Liter wasserfreiem Methanol werden 0,72 kg granuliertes Ätznatron gegeben und gerührt bis alles in Lösung gegangen ist. Dazu werden 4,34 kg Bis-2-ethylhexylamin gegeben und innerhalb von zwei Stunden 1,1 Liter Schwefelkohlenstoff eingetragen. Es wird noch 3 Stunden nachgerührt und anschließend eine Lösung von 1,19 kg Wolframhexachlorid in 3 Liter wasserfreiem Methanol zugegeben und 3 Stunden zur Reaktion gebracht. Vom Niederschlag wird abfiltriert und aus dem Filtrat wird das Lösungsmittel abdestilliert. Der Rückstand, ein dunkelbraunes pastöses Produkt, wird bei 50°C im Vakuum getrocknet.
- Ausbeute:: 85 % der Theorie
- Gehalt:: über Stickstoffbestimmung―99,6 %

## Patentansprüche

1. Wolfram-hexa-(dialkyldithiocarbamate) der allgemeinen Formel I 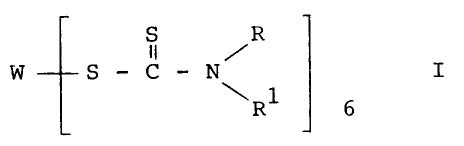 in der R und R¹ gleiche oder verschiedene, verzweigte oder unverzweigte, gesättigte oder ungesättigte Alkylreste mit 1 - 18 Kohlenstoffatomen bedeuten.

2. Wolfram-hexa(di-amyldithiocarbamat) nach Anspruch 1.

3. Wolfram-hexa(di-2-ethylhexyldithiocarbamat) nach Anspruch 1.

4. Verfahren zur Herstellung von Wolfram-hexa-(dialkyldithiocarbamaten) der allgemeinen Formel I, dadurch gekennzeichnet, daß man 1 Mol Wolframhexachlorid mit 6 Mol eines Dialkyl-dithiocarbamat der allgemeinen Formel II 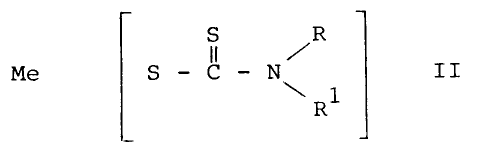 in der R und R¹ die Bedeutung laut Anspruch 1 haben und Me für Natrium, Kalium oder Ammonium steht, umsetzt, das gebildete Natrium- Kalium- oder Ammoniumchlorid abtrennt und das Wolfram- hexa-(dialkyldithiocarbamat) isoliert.

5. Verfahren nach Anspruch 4, dadurch gekennzeichnet, daß wasserfreie Lösungsmittel wie Methanol, Ethanol, Butanol, Isopropanol, Dioxan, Tetrahydrofuran oder Ethylenglykolmonoalkylether eingesetzt werden.

6. Verfahren nach einem der Ansprüche 4 oder 5, dadurch gekennzeichnet, daß die Umsetzung bei Temperaturen von 10 - 90°C, vorzugsweise bei 20 - 30°C erfolgt.

7. Verwendung der Wolfram-hexa-(dialkyldithiocarbamate) nach Anspruch 1 bis 3 als Additive für Hydraulik- oder Getriebedaueröle.

## Claims

1. Tungsten-hexa-(dialkyldithiocarbamates) conforming to the general formula I below: 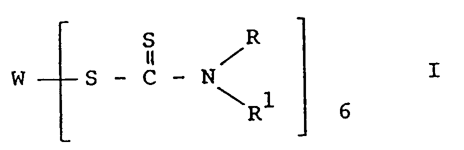 in which R and R¹ denote identical or different, branched or unbranched, saturated or unsaturated alkyl radicals with between 1 and 18 carbon atoms.

2. Tungsten-hexa-(di-amyldithiocarbamate) according to Claim 1.

3. Tungsten-hexa-(di-2-ethylhexyldithiocarbamate) according to Claim 1.

4. Process for the production of tungsten-hexa-(dialkyldithiocarbamates) conforming to the general formula I, characterized in that 1 mole of tungsten hexachloride is reacted with 6 moles of a dialkyldithiocarbamate conforming to the general formula II below: 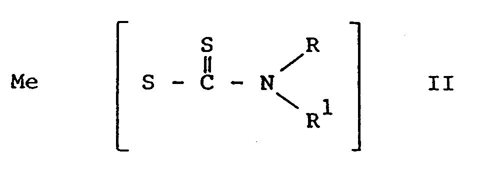 in which R and R¹ denote the same as in Claim 1 and Me denotes sodium, potassium or ammonium, the sodium, potassium or ammonium chloride so formed is separated off and the tungsten-hexa-(dialkyldithiocarbamate) is then isolated.

5. Process according to Claim 4, characterized in that water-free solvents such as methanol, ethanol, butanol, isopropanol, dioxane, tetrahydrofuran or ethyleneglycol-monoalkylether are used.

6. Process according to either of Claims 4 or 5, characterized in that the reaction takes place at temperatures of 10°C to 90°C, and preferably between 20°C to 30°C.

7. The utilization of tungsten-hexa-(dialkyldithiocarbamates) according to Claims 1 to 3, as additives for hydraulic or long-life transmission lubricant oils.

## Revendications

1. Hexa-(dialkyldithiocarbamates) de tungstène de formule générale I 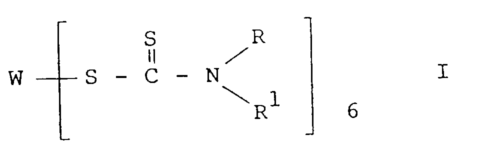 dans laquelle R et R¹ représentent des restes alkyle saturés ou non saturés, linéaires ou ramifiés, égaux ou différents ayant 1 à 18 atomes de carbone.

2. L'hexa(diamyldithiocarbamte) de tungstène suivant la revendication 1.

3. L'hexa(di-2-éthylhexyldithiocarbamate) de tungstène suivant la revendication 1.

4. Procédé de production d'hexa-(dialkyldithiocarbamates) de tungstène de formule générale I, caractérisé en ce qu'on fait réagir une mole d'hexachlorure de tungstène avec 6 moles d'un dialkyldithiocarbamate de formule générale II 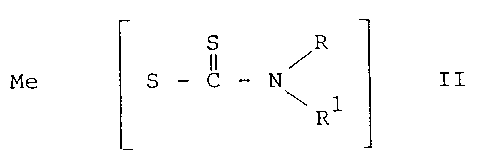 dans laquelle R et R¹ ont la définition indiquée dans la revendication 1 et Me représente le sodium, le potassium ou l'ion ammonium, on sépare le chlorure de sodium, de potassium ou d'ammonium formé et on isole l'hexa-(dialkyldithiocarbamate) de tungtène.

5. Procédé suivant la revendication 4, caractérisé en ce qu'on utilise des solvants anhydres tels que le méthanol, l'éthanol, le butanol, l'isopropanol, le dioxanne, le tétrahydrofuranne ou un éther monoalkylique d'éthylèneglycol.

6. Procédé suivant l'une des revendications 4 ou 5, caractérisé en ce que la réaction a lieu à des températures de 10 à 90°C, de préférence à 20 - 30°C.

7. Utilisation des hexa-(dialkyldithiocarbamates) de tungstène suivant les revendications 1 à 3 comme additifs pour huiles hydrauliques ou huiles pour engrenages.
